Europäisches Patentamt

European Patent Office

Office européen des brevets

(19)

(11) Veröffentlichungsnummer: **0 204 986**
**B1**

(12) **EUROPÄISCHE PATENTSCHRIFT**

(45) Veröffentlichungstag der Patentschrift:
04.04.90

(51) Int. Cl.⁴: **A61J 15/00**

(21) Anmeldenummer: **86106588.6**

(22) Anmeldetag: **15.05.86**

(54) **Nasenolive.**

(30) Priorität: **17.05.85 DE 3517747**

(43) Veröffentlichungstag der Anmeldung:
**17.12.86 Patentblatt 86/51**

(45) Bekanntmachung des Hinweises auf die Patenterteilung:
**04.04.90 Patentblatt 90/14**

(84) Benannte Vertragsstaaten:
**AT BE CH DE FR GB IT LI LU NL SE**

(56) Entgegenhaltungen:
DE-B- 1 029 127
GB-A- 1 229 438
US-A- 2 335 936

(73) Patentinhaber: **Fresenius AG, Gluckensteinweg 5,
D-6380 Bad Homburg v.d.H.(DE)**

(72) Erfinder: **Milewski, Christian, Dr. med.,
14-Nothelferstrasse 26, D-6500 Mainz-Gonsenheim(DE)**
Erfinder: **Welzer, Peter, Sartoriusring 27,
D-6500 Mainz-Finthen(DE)**

(74) Vertreter: **Görtz, Dr. Fuchs, Dr. Luderschmidt
Patentanwälte,
Abraham-Lincoln-Strasse 7 Postfach 46 60,
D-6200 Wiesbaden(DE)**

## Beschreibung

Die Erfindung betrifft eine Vorrichtung zur Applizierung von Flüssigkeiten durch die Nase gemäß dem Oberbegriff des Anspruchs 1 oder 4.

Derartige Vorrichtungen sind beispielsweise aus dem Bereich der künstlichen Ernährung bekannt. Hierzu wird dem Patienten üblicherweise, der häufig über sehr lange Zeiträume auf eine künstliche Ernährung angewiesen ist, ein Sondenschlauch durch die Nase eingeführt, so daß sich das distale Ende des Sondenschlauches im Magenbereich befindet, während das proximale Ende des Sondenschlauches von außen zugänglich außerhalb der Nase angeordnet ist. Hierzu wird dieses Ende bei bekannten Vorrichtungen auf der Nase oder benachbarten Gesichtspartien mittels Heftpflaster befestigt, da das proximale Ende lagegesichert sein muß, um das Zurück- oder Herausrutschen während der ernährungsfreien Zeit zu verhindern.

Hierbei ist jedoch nachteilig, daß bei den bekannten Vorrichtungen das proximale Ende des Sondenschlauches aufgrund seiner Befestigungsart sichtbar ist. Dies bringt insbesondere für Patienten, die aufgrund ihrer Krankheit langwährende künstliche Ernährung benötigen, den Nachteil mit sich, daß ihre Abhängigkeit von dieser Ernährungsart und damit das Bestehen ihrer Krankheit nach außen hin offen zutage tritt, was bei einer Vielzahl von Patienten wiederum zu negativen psychischen Auswirkungen führt. Darüber hinaus stellt die sichtbare Befestigung des proximalen Endes des Sondenschlauches eine nicht zu vernachlässigende Beeinträchtigung des äußeren Erscheinungsbildes des Patienten dar.

Aus dem Katalog der Firma Rüsch Chirurgie III/3, 2. Auflage, 1977, Seite 14 und Seite 36 ist eine Ernährungssonde bzw. ein pneumatischer Nasentubus bekannt. Die hier gezeigte Ernährungssonde weist die vorstehend bekannten Probleme auf, d.h. sie ist aus der Nase des Patienten geführt und stellt somit eine sichtbare Beeinträchtigung des Patienten dar.

Der pneumatische Nasentubus besteht aus einem die Nasenhöhle ausfüllenden Körper, von dem sich ein Anschlußstutzen zum Nasenloch hin erstreckt, der aus dem Nasenloch herausragt, somit also ebenfalls eine sichtbare Beeinträchtigung des Patienten darstellt.

Zur Lösung der beim Einsatz von Ernährungssonden auftretenden Probleme ist das operative Einsetzen der Schlauchsonde im unmittelbaren Bereich des Magens vorgeschlagen worden, was sich jedoch in der Praxis nicht durchsetzen konnte, da eine Vielzahl von Patienten den erforderlichen Eingriff scheuen, bei einigen der Eingriff nicht möglich ist bzw. zu zusätzlichen unerwünschten Risiken führt.

Aus der GB-A 1 229 438 ist eine Vorrichtung zur Applizierung von Flüssigkeiten durch die Nase bekannt, bei der der Sondenschlauch nicht nur im Nasenvorhof, sondern auch in der Nasenhöhle festgelegt ist. Die bekannte Vorrichtung füllt nicht den gesamten Nasenvorhof aus, da Raum für die freie Beatmung zur Verfügung stehen muß. Zur Fixierung der Vorrichtung ist daher ein Bügel 3 vorgesehen, der außen an der Nase anliegt und infolge seiner Sichtbarkeit das Wohlbefinden des Patienten einschränkt.

Es ist daher Aufgabe der Erfindung, eine Vorrichtung zur Applizierung von Flüssigkeiten durch die Nase mittels eines Sondenschlauches der im Oberbegriff des Anspruchs 1 oder 4 umrissenen Gattung zu schaffen, die die Vermeidung einer Beeinträchtigung des Patienten durch die sichtbare Fixierung des proximalen Endes des Sondenschlauches ermöglicht.

Diese Aufgabe wird durch die kennzeichnenden Merkmale des Anspruchs 1 oder des Anspruchs 4 gelöst.

Die Unteransprüche haben vorteilhafte Weiterbildungen der Erfindung zum Inhalt.

Zur Verbesserung der Trageeigenschaften ist die Außenkontur des Lagerteiles an die Innenkontur des Nasenvorhofes angepaßt, wobei es möglich ist, eine Kontur herzustellen, die einer Vielzahl von unterschiedlich ausgebildeten Nasenvorhofformen gerecht wird. Selbstverständlich ist es natürlich auch möglich, das Lagerteil jeweils individuell anzupassen.

Zur Verbesserung der Lagesicherung ist das Ende des Sondenschlauches mit einem Anschlußteil versehen, das eine Stützfläche aufweist, wobei das Anschlußteil in einer vorteilhaften Ausführungsform als handelsüblicher Sondenschlauch-Konnektor ausgebildet sein kann, was den Vorteil mit sich bringt, daß dies eine besonders kostengünstige Lösung darstellt.

Das Anschlußteil wird in dem in den Nasenvorhof einführbaren Lagerteil angeordnet, wodurch das Benutzen der erfindungsgemäßen Vorrichtung für den Patienten angenehmer und leichter gemacht wird.

Hierzu kann sich das Lagerteil wiederum an der Nasenklappe abstützen, während sich die Stützfläche des Anschlußteiles an einer Lagerfläche des Lagerteiles abstützen kann, was eine weitere Verbesserung der Benutzungseigenschaften der erfindungsgemäßen Vorrichtung mit sich bringt.

Da die Lagerfläche des Lagerteiles eine harte Oberfläche aufweisen muß, um einen dauerhaft sicheren Sitz gegen Herabrutschen des Sondenschlauches zu gewährleisten, ergibt sich bei Anordnung dieser Lagerfläche in einem Hohlraum des Lagerteiles der Vorteil, daß die Naseninnenwand nicht mit der Lagerfläche in Berührung kommen kann, wodurch Verletzungen und Unannehmlichkeiten für den Patienten vermieden werden.

Weist der Hohlraum an zwei Seiten Ausnehmungen auf, die im in den Nasenvorhof eingeführten Zustand zum einen zum Nasenloch und zum anderen zur Nasenklappe hinweisen, ergibt sich der Vorteil, daß den natürlichen anatomischen Gegebenheiten in vollem Umfange Rechnung getragen ist, so daß bei einer entsprechend großen Ausbildung der Ausnehmungen genügend Freiraum vorhanden ist, damit eine Behinderung des Patienten beim Atmen verhindert werden kann.

Weist der Hohlraum des Lagerteiles einen Teilbereich auf, innerhalb dessen das gesamte Anschlußteil in der applizierungsfreien Zeit unverlierbar gelagert werden kann, ergibt sich der Vorteil, daß jegliche Verletzungsgefahr und Unannehmlichkeiten für den Patienten für die Zeit behoben sind, in der das proximale Ende des Sondenschlauches innerhalb des Nasenvorhofes angeordnet ist.

Zur Applizierung der Flüssigkeit, wie beispielsweise bei der künstlichen Ernährung, wird das proximale Ende des Sondenschlauches lediglich aus der Nase entnommen, um mit dem Flüssigkeitsbehälter oder einem Sondenverlängerungsstück verbunden zu werden. In der applizierungsfreien Zeit hingegen ist das proximale Ende innerhalb des Nasenvorhofes angeordnet und damit unsichtbar, so daß die Abhängigkeit des Patienten, insbesondere bei langwährenden Dauerapplizierungen, wie einer ständigen künstlichen Ernährung, nicht mehr offen zutage tritt. Zudem ist das proximale Ende des Sondenschlauches in der applizierungsfreien Zeit lagegesichert, so daß nicht die Gefahr besteht, daß es durch die Nase nach hinten in den Körper rutscht oder aus der Nase völlig herausrutscht, wobei dem Patienten trotzdem jegliche Bewegungsfreiheit gegeben ist.

Durch eine entsprechende Bearbeitung der Oberfläche des Lagerteiles kann deren Rauhigkeit auf ein Maß reduziert werden, daß die Trageeigenschaften auf einen optimalen Wert verbessert werden können, wobei das Lagerteil aus physiologisch verträglichem Material besteht.

Zur Vermeidung des Rückfließens des Mageninhalts durch das proximale Ende des Sondenschlauches kann dessen Anschlußteil verschließbar ausgebildet sein, wozu vorteilhafterweise ein abnehmbarer Verschlußstopfen verwendet werden kann.

Bei der Lösung gemäß den Ansprüchen 4 bis 7 ergeben sich insbesondere noch Vorteile bei der Fertigung der erfindungsgemäßen Vorrichtung, da diese Konstruktion äußerst einfach und kostengünstig hergestellt werden kann.

Weitere Einzelheiten, Merkmale und Vorteile der Erfindung ergeben sich aus nachfolgender Beschreibung von Ausführungsbeispielen anhand der Zeichnung.

Es zeigen:

Fig. 1 eine Schnittdarstellung eines Lagerteils einer ersten Ausführungsform der erfindungsgemäßen Vorrichtung,

Fig. 2 eine Ansicht des Lagerteiles aus Richtung des Pfeiles II in Fig. 1,

Fig. 3 den Bereich des proximalen Endes eines Sondenschlauches der erfindungsgemäßen Vorrichtung mit einem Anschlußteil,

Fig. 4 eine der Fig. 1 entsprechende Darstellung des Lagerteiles, in dem das proximale Ende des Sondenschlauches mit dem Anschlußteil gemäß Fig. 3 angeordnet ist, und die der Anordnung in der applizierungsfreien Zeit entspricht,

Fig. 5 eine der Fig. 2 entsprechende Darstellung des Lagerteiles gemäß den Fig. 1 und 4 mit darin abgestütztem Anschlußteil gemäß Fig. 3, wobei diese Anordnung während der Applizierung vorliegt, und

Fig. 6 eine den Fig. 1 und 4 entsprechende Darstellung eines Lagerteiles einer zweiten Ausführungsform der erfindungsgemäßen Vorrichtung.

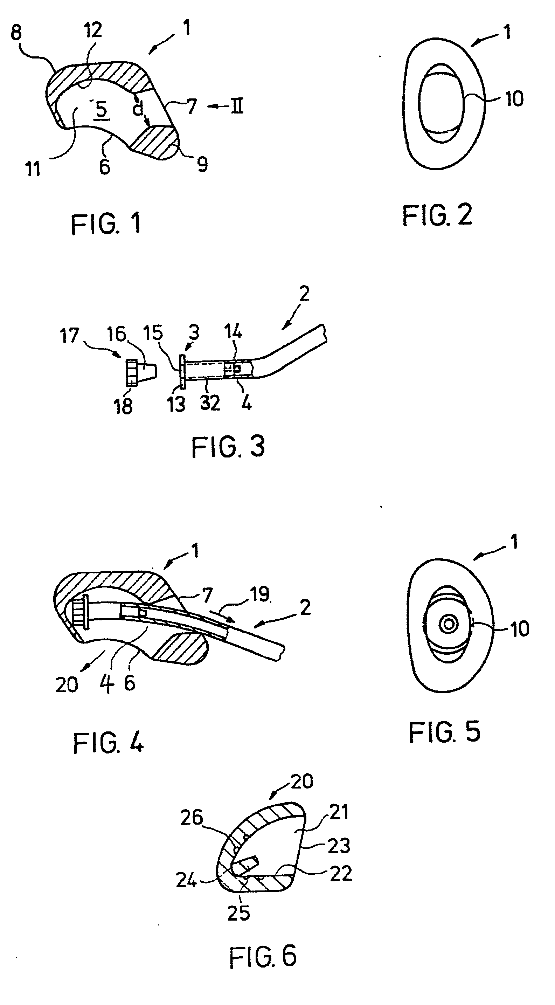

Die gemäß den Fig. 1 bis 5 dargestellte erste Ausführungsform einer erfindungsgemäßen Vorrichtung zur Applizierung von Flüssigkeiten durch die Nase weist ein Lagerteil 1, einen Sondenschlauch 2 und im Beispielsfalle ein Anschlußteil 3 auf. Hierbei ist der Sondenschlauch lediglich im Bereich seines proximalen Endes 4 dargestellt. Das distale Ende des Sondenschlauches 2 ist im eingeführten Zustand an der Stelle des Körperinneren angeordnet, zu der die jeweilige Flüssigkeit zugeführt werden soll. Im Falle der künstlichen Ernährung, von der im weiteren beispielhaft ausgegangen wird, ist das distale Ende im Bereich des Magens angeordnet.

Das gemäß Fig. 1 dargestellte Lagerteil 1 weist im Beispielsfalle einen Hohlraum 5 auf, der an zwei Seiten offen ausgebildet ist, wozu jeweils eine Ausnehmung 6 bzw. 7 vorgesehen ist. Hierbei entspricht die Ausnehmung 6 derjenigen, die im in den Nasenvorhof eingeführten Zustand des Lagerteiles 1 dem Nasenloch benachbart angeordnet ist. Die Ausnehmung 7 weist dementsprechend in diesem Zustand zur Nasenklappe hin. Dies bedeutet weiterhin, daß der gemäß der in Fig. 1 gewählten Darstellung linke obere Bereich 8 des Lagerteiles 1 im in den Nasenvorhof eingeführten Zustand sich an dessen nach vorne weisenden oberen Innenbereich abstützt, während der rechts unten liegende Teilbereich 9 des Lagerteiles im hinteren unteren Bereich des Nasenvorhofes angeordnet ist.

Das Lagerteil 1 weist ferner eine gemäß den Fig. 2 und 5 dargestellte Lagerfläche 10 auf, die innerhalb des Hohlraumes 5 im Bereich des in Fig. 1 mit d bezeichneten Querschnittes angeordnet ist, und die gemäß den Fig. 2 und 5 eine ellipsenförmige Ausbildung aufweist. Diese Ausbildung bringt den Vorteil mit sich, daß die Lagerfläche 10 zum einen eine sichere Abstützung im Bereich des kleineren Ellipsendurchmessers bietet, zum anderen jedoch im Bereich des oberen Ellipsendurchmessers, beispielsweise bei kreisförmiger Ausbildung der an die Lagerfläche 10 angelegten Gegenfläche, genug Freiraum läßt, um Durchtrittswege für die Atemluft freizulassen.

Weiterhin weisen die Ausnehmungen 6 und 7 ebenfalls genügend große Dimensionen auf, um den Durchgang von Atemluft zu ermöglichen. Ferner ist die Größe der Ausnehmung 7 so gewählt, daß dem proximalen Ende 4 des Sondenschlauches 2 im in das Lagerteil 1 eingesetzten Zustand genügend Bewegungsfreiheit gegeben wird, um den Bewegungen des Patienten folgen zu können. Die Ausnehmung 6 weist eine solche Größe auf, daß ein Herausnehmen des proximalen Endes 4 durch diese Ausnehmung 6 hindurch auch mit aufgesetztem Anschlußteil 3 möglich ist.

Durch die Anordnung der Lagerfläche 10 innerhalb des Hohlraumes 5 ist gewährleistet, daß die harte Oberfläche der Lagerfläche 10, die zur Gewährleistung einer sicheren Rückhaltefunktion erforderlich ist, keinerlei Verletzungen oder Unannehmlichkeiten für den Patienten hervorrufen kann.

Das Lagerteil 1 ist ferner hinsichtlich seiner Außenkontur an die Innenkontur des Nasenvorhofes angepaßt, woraus sich die gemäß den Fig. 2 und 5 ersichtliche olivenähnliche Ausbildung ergibt, weswegen das Lagerteil 1 auch schlagwortartig als Nasenolive bezeichnet werden kann.

Der Hohlraum 5 des Lagerteiles 1 weist ferner einen Teilbereich 11 auf, der von einem abgerundeten Wandbereich 12 des Hohlraumes 5 begrenzt wird. Die Funktion dieses Teilbereiches 11 wird im folgenden näher erläutert.

Das gemäß Fig. 3 dargestellte Anschlußteil 3 weist einen zylinderförmigen Hauptteil 32 auf, der an einer Seite mit einer im Beispielsfalle kreisringförmig ausgebildeten Stützfläche 13 versehen ist. Am gegenüberliegenden Ende weist das Anschlußteil 3 einen Einsteckzapfen 14 auf, über den das proximale Ende 4 des Sondenschlauches 2 gestülpt werden kann. Das Anschlußteil 3 ist innen hohl ausgebildet, so daß eine Strömungsverbindung zwischen dem Anschlußteil 3 und dem aufgesteckten Sondenschlauch 2 hergestellt werden kann. Die in Fig. 3 dargestellte Ausführungsform des Anschlußteiles 3 stellt einen handelsüblichen Sondenschlauch-Konnektor dar, dessen flanschförmige Stützfläche 13 eine Ausnehmung 15 aufweist, in die ein Zapfen 16 eines Verschlußstopfens 17 eingeführt werden kann, womit das offene Ende des Anschlußteiles 3 verschlossen werden kann. Der Verschlußstopfen 17 weist weiter ein gerändeltes Stopfenende 18 auf, mittels dessen der Verschlußstopfen 17 in die Ausnehmung 15 eingeführt bzw. aus ihr herausgezogen werden kann.

Gemäß Fig. 4 ist verdeutlicht, auf welche Art und Weise das mit dem Verschlußstopfen 17 versehene Anschlußteil 3 im Lagerteil 1 angeordnet werden kann, wenn die Vorrichtung während der ernährungsfreien Zeit nicht in Gebrauch ist und das Lagerteil 1 in den Nasenvorhof eingeführt ist.

Hierzu wird der Bereich der Stützfläche 13, auf dem der Verschlußstopfen 17 angeordnet ist, in den Teilbereich 11 des Hohlraumes 5 eingeführt, wobei sich das Anschlußteil 3 mit dem Verschlußstopfen 17 und der Stützfläche 13 an der Innenwandfläche 12 des Teilbereiches 11 abstützt. Die zylinderförmige Hauptteil 32 und der Anschlußzapfen 14 des Anschlußteiles 3 befinden sich dann in dem gemäß der in Fig. 4 dargestellten unteren Bereich des Hohlraumes 5, wobei durch die abgerundete Ausbildung der Innenwandfläche 12 dem Anschlußteil 3 eine genügend große Beweglichkeit verbleibt, so daß sich das proximale Ende des Sondenschlauches 2 innerhalb der genügend groß ausgebildeten Ausnehmung 7 bewegen kann, so daß es den Bewegungen des Patienten, die die Bewegungen des Sondenschlauches 2 hervorrufen, folgen kann.

Soll eine Applizierung von Flüssigkeit, wie bei der künstlichen Ernährung, durchgeführt werden, kann das Lagerteil 1 durch Fingerdruck auf den Nasenflügel aus dem Nasenvorhof entfernt werden, wobei es das proximale Ende 4 des Sondenschlauches 2 mitnimmt. Danach kann das Anschlußteil 3 durch Ziehen am Sondenschlauch 2 in Richtung des Pfeiles 19 aus dem Teilbereich 11 des Hohlraumes 5 herausgezogen werden und durch Drücken in Richtung des Pfeiles 20 durch die Ausnehmung 6 hindurch aus dem Lagerteil 1 herausgedrückt werden. Danach kann der Verschlußstopfen 17 entnommen werden und das Anschlußteil 3 mit dem entsprechenden Gegenstück der Flüssigkeitszuführleitung verbunden werden. Ist die Zuführung der Flüssigkeit beendet, kann das Anschlußteil wieder verschlossen werden und in die in Fig. 4 dargestellte Lage innerhalb des Lagerteiles 1 zurück überführt werden. In diesem Zustand wird das Lagerteil 1 wieder in den Nasenvorhof eingeführt, in dem es unsichtbar angeordnet ist und in dem das proximale Ende 4 des Sondenschlauches 2 lagegesichert fixiert ist.

Die Lagefixierung gegen ein unerwünschtes Herausrutschen des Sondenschlauches 2 in Richtung des Pfeiles 19 wird durch die Anlage der Stützfläche 13 an der Lagerfläche 10 des Lagerteiles 1 gewährleistet, die in Fig. 5 verdeutlicht ist. Hieraus ist ersichtlich, daß die kreisförmige Stützfläche 13 in den gestrichelt dargestellten Bereichen an der Lagerfläche 10 anliegt und somit ein Zurückgleiten des Sondenschlauches 2 in das Körperinnere verhindert wird. Dabei kann sich jedoch der Sondenschlauch 2 in der Längsrichtung der Ausnehmung 7 frei bewegen, was ebenfalls aus Fig. 5 hervorgeht und was zuvor bereits erläutert worden ist.

Gemäß Fig. 6 ist eine andere Ausbildung eines Lagerteiles 20 dargestellt, das einen Hohlraum 21 aufweist, der von einer Innenwandfläche 22 des Lagerteiles 20 begrenzt wird. Der Hohlraum 21 ist einseitig über eine Ausnehmung 23 offen und weist an der der Ausnehmung 23 gegenüberliegenden Lagerfläche 24 einen Führungszapfen 25 auf. Im Bereich dieses Führungszapfens 25 ist in der Innenwandfläche 22 im Bereich der Lagerfläche 24 ein Gewinde 26 angeordnet, das mit der Stützfläche 13 des Anschlußteiles 3 zusammenwirken kann. Damit kann das Anschlußteil 3 und damit das distale Ende 4 des Sondenschlauches 2 in das Lagerteil 20 durch Drehung des Anschlußteiles 3 eingebracht und in diesem durch Wechselwirkung der Stützfläche 13 mit dem Gewinde 26 festgelegt werden. Der Führungszapfen 25 unterstützt dabei die Lagerung des Anschlußteiles 3. Die sonstige Ausbildung des Lagerteiles 20 bezüglich seiner Außenkontur, Oberflächenstruktur und seiner Funktion und Anordnung in dem Nasenvorhof entspricht derjenigen der Ausführung gemäß den Fig. 1 bis 5, so daß die Erläuterung bezüglich dieser Punkte auch für die Ausführungsform gemäß Fig. 6 zutrifft. Weiterhin kann auch das gemäß Fig. 3 dargestellte Anschlußteil 3 zusammen mit dem Lagerteil 20 verwendet werden.

Hinzuzufügen ist, daß die Stützfläche 13 nicht nur eine zusammenhängende Kreisform, sondern vielmehr auch mehrere radial abstehende, voneinander getrennte Stützelemente aufweisen kann, die gegebenenfalls als Schraubelemente dienen.

**Patentansprüche**

1. Vorrichtung zur Applizierung von Flüssigkeiten durch die Nase
   – mit einem Lagerteil (1), das der Kontur des Nasenvorhofes angepaßt ist und welches einen Hohlraum (5) aufweist, der an zwei Seiten über jeweils eine Ausnehmung (6, 7) offen ist, wobei im eingeführten Zustand die eine, vordere Ausnehmung (6) zum Nasenloch, während die andere, hintere Ausnehmung (7) zur Nasenklappe hinweist, und
- mit einem Sondenschlauch (2), der ein distales und ein proximales Ende aufweist, wobei das Lagerteil (1) und der Sondenschlauch (2) zweistückig sind, dadurch gekennzeichnet, daß
- das Lagerteil zur ausschließlichen Aufnahme im Nasenvorhof bestimmt und
- die äußere Form des Lagerteils (1) der ganzen Kontur des Nasenvorhofs anschmiegend angepaßt ist,
- das proximale Ende (4) des Sondenschlauches (2) mit einem Anschlußteil (3) versehen ist, das eine Stützfläche (13) aufweist, und
- der Hohlraum (5) des Lagerteiles einen Teilbereich (11, 24) aufweist, innerhalb dessen in der applizierungsfreien Zeit das Anschlußteil (3), die hintere Ausnehmung durchdringend, lagegesichert, aber abnehmbar fixiert ist.

2. Vorrichtung nach Anspruch 1, dadurch gekennzeichnet, daß die Ausnehmungen (6, 7) eine Größe aufweisen, die auch bei in den Nasenvorhof eingeführtem Lagerteil (1) und darin angeordnetem Anschlußteil (3) genügend große Durchtrittswege für Atemluft gewährleistet.

3. Vorrichtung nach einem der Ansprüche 1 oder 2, dadurch gekennzeichnet, daß der Teilbereich (11) im Hohlraum (5), angepaßt an die Stützfläche (13), von einem abgerundeten Wandbereich (12) des Hohlraumes (5) begrenzt ist und daß der Hohlraum zusätzlich eine Lagerfläche (10) aufweist, an der sich die Stützfläche (13) zur Verhinderung des Zurückgleitens des Sondenschlauches (2) in das Körperinnere zusätzlich abstützt.

4. Vorrichtung zur Applizierung von Flüssigkeiten durch die Nase
- mit einem Lagerteil (20), das der Kontur des Nasenvorhofes angepaßt ist und welches einen Hohlraum (21) aufweist, der an einer Seite über eine Ausnehmung (23) offen ist, wobei im eingeführten Zustand die Ausnehmung zur Nasenklappe hinweist, und
- mit einem Sondenschlauch (2), der ein distales und ein proximales Ende aufweist, wobei das Lagerteil (20) und der Sondenschlauch (2) zweistückig sind, dadurch gekennzeichnet, daß
- das Lagerteil (20) zur ausschließlichen Aufnahme im Nasenvorhof bestimmt und
- die äußere Form des Lagerteils (20) der ganzen Kontur des Nasenvorhofs anschmiegend angepaßt ist,
- das proximale Ende (4) des Sondenschlauches (2) mit einem Anschlußteil (3) versehen ist, das eine Stützfläche (13) aufweist,
- der Hohlraum (21) des Lagerteiles (20) einen Teilbereich (24) aufweist, innerhalb dessen in der applizierungsfreien Zeit das Anschlußteil (3), die zur Nasenklappe hinweisende Ausnehmung durchdringend, lagegesichert, aber abnehmbar fixiert ist, und
- im Teilbereich (24) in dem zugehörigen Abschnitt der Innenwandfläche (Lagerfläche 24) ein Gewinde (26) ausgebildet ist, das mit der Stützfläche (13) des Anschlußteiles (3) in Wirkeingriff bringbar ist.

5. Vorrichtung nach Anspruch 4, dadurch gekennzeichnet, daß an der der hinteren Ausnehmung (23) des Hohlraums (21) gegenüberliegenden Lagerfläche (24) ein Führungszapfen (25) für eine zugeordnete Ausnehmung des Anschlußteils (3) angeordnet ist.

6. Vorrichtung nach einem der Ansprüche 1–5, dadurch gekennzeichnet, daß das Anschlußteil (3) als konventioneller Sondenschlauchkonnektor ausgebildet ist, dessen Stützfläche (13) flanschförmig ausgebildet ist.

7. Vorrichtung nach einem der Ansprüche 1–6, dadurch gekennzeichnet, daß die Oberfläche des Lagerteils (1, 20) eine geringe Rauhigkeit aufweist und das Lagerteil (1, 20) aus physiologisch verträglichem Material besteht.

## Claims

1. A device for administering fluids through the nose, comprising
- a bearing portion (1) conforming to the contour of the nasal auricle and exhibiting a cavity (5) open at two ends over respectively one recess (6, 7), with the front recess, in the introduced condition, pointing to the nostril and the rear recess (7) pointing to the nasal valve, and
- a probe tube (2) exhibiting a distal and a proximal end, with the bearing portion (1) and the probe tube (2) being of a bipartite configuration, characterized in that
- the bearing portion is intended for the exclusive reception in the nasal auricle, and,
- the outer shape of the bearing portion (1), throughout the nasal auricle, conforms to the contour thereof,
- the proximal end (4) of the probe tube (2) has a connecting piece (3) exhibiting a supporting face (13), and
- the cavity (5) of the bearing portion exhibits a partial area (11, 24) within which, during non-administering times, the connecting piece (3) is detachably fixed while passing through the rear recess and being locked in position.

2. A device according to claim 1, characterized in that the recesses (6, 7) are sized to insure sufficiently large passageways for respiration air even with bearing portion (1) introduced into the nasal auricle and with connecting piece (3) located therein.

3. A device according to any one of claims 1 or 2, characterized in that the partial area (11) within the cavity (5), in adaptation to the supporting face (13), is confined by a rounded-off wall area (12) of the cavity (5), and that the cavity, in addition, exhibits a bearing face (10) on which the supporting face (13) is additionally supported to prevent the probe tube (2) from sliding back into the interior of the body.

4. A device for administering fluids through the nose, comprising
- a bearing portion (20) conforming to the contour of the nasal auricle and exhibiting a cavity (21) open at one end through a recess (23), with the recess, in the introduced condition, pointing to the nasal valve, and
- a probe tube (2) exhibiting a distal and a proximal end, with the bearing portion (20) and the probe hose (2) being of a bipartite configuration, characterized in that
- the bearing portion (20) is intended for the exclusive reception in the nasal auricle, and
- the outer shape of the bearing portion (20), throughout the nasal auricle, conforms to the contour thereof,
- the proximal end (4) of the probe tube (2) is provided with a connecting portion (3) exhibiting a supporting face (13),
- the cavity (21) of the bearing portion (20) exhibits a partial area (24) within which, during non-administering times, the connecting portion (3) is detachably fixed while passing through the recess facing the nasal valve and being locked in position, and
- a thread (26) is formed within the partial area (24), in the associated section of the internal wall face (bearing face 24), which thread can be placed in cooperative engagement with the supporting face (13) of the connecting portion (3).

5. A device according to claim 4, characterized in that disposed on the bearing face (24) opposite the rear recess (23) of the cavity (21) is a guiding pin (25) for an associated recess of the connecting portion (3).

6. A device according to any one of claims 1 to 5, characterized in that the connecting portion (3) is in the form of a conventional probe tube connector the supporting faces (13) of which are of a flange-type configuration.

7. A device according to any one of claims 1 to 6, characterized in that the surface of the bearing portion (1, 20) is of a minor coarseness and that the bearing portion (1, 20) consists of a physiologically tolerable material.

**Revendications**

1.- Dispositif pour l'introduction de liquides par le nez, comportant :
- une pièce de positionnement (1), qui est adaptée au contour de la partie antérieure de la fosse nasale et qui présente un creux (5) ouvert des deux côtés chaque fois par une lumière (6, 7), étant entendu que dans l'état introduit en place, la première lumière antérieure (6) est dirigée vers la narine, tandis que l'autre lumière postérieure (7) est dirigée vers la cloison nasale, et
- une sonde souple (2), qui comporte une extrémité distale et une extrémité proximale, la pièce de positionnement (1) et la sonde souple (2) constituant deux pièces, caractérisé en ce que :
- la pièce de positionnement est destinée à être reçue exclusivement dans la partie antérieure de la fosse nasale, et
- la forme extérieure de la pièce de positionnement (1) est adaptée étroitement à l'ensemble du contour de la partie antérieure de la fosse nasale;

- l'extrémité proximale (4) de la sonde souple (2) est pourvue d'une pièce de raccordement (3) qui présente une surface d'appui (13), et
- le creux (5) de la pièce de positionnement comporte une zone partielle (11, 24) à l'intérieur de laquelle, pendant la période de non-introduction du liquide, la pièce de raccordement (3), traversant la lumière postérieure, est fixée, assujettie en place mais amovible.

2.- Dispositif suivant la revendication 1, caractérisé en ce que les lumières (6, 7) présentent une dimension qui garantit, même lorsque la pièce de positionnement (1) est introduite dans la partie antérieure de la fosse nasale et que la pièce de raccordement (3) y est installée, des trajets de passage suffisamment grands pour l'air de respiration.

3.- Dispositif suivant la revendication 1 ou 2, caractérisé en ce que la zone partielle (11) dans le creux (5), adaptée à la surface d'appui (13), est délimitée par une zone de paroi courbe (12) du creux (5) et le creux présente, en outre, une surface de positionnement (10) sur laquelle la surface d'appui (13) prend également appui pour empêcher la sonde souple (2) de reglisser à l'intérieur du corps.

4.- Dispositif pour l'introduction de liquides par le nez comportant :
- une pièce de positionnement (20) qui est adaptée au contour de la partie antérieure de la fosse nasale et qui présente un creux (21) ouvert d'un côté par une lumière (23), étant entendu que, dans l'état introduit en place, la lumière est dirigée vers la cloison nasale, et
- une sonde souple (2), qui présente une extrémité distale et une extrémité proximale, la pièce de positionnement (20) et la sonde souple (2) constituant deux pièces, caractérisé en ce que :
- la pièce de positionnement (20) est destinée à être reçue exclusivement dans la partie antérieure de la fosse nasale, et
- la forme extérieure de la pièce de positionnement (20) est adaptée étroitement à l'ensemble du contour de la partie antérieure de la fosse nasale;
- l'extrémité proximale (4) de la sonde souple (2) est pourvue d'une pièce de raccordement (3) qui présente une surface d'appui (13);
- le creux (21) de la pièce de positionnement (20) comporte une zone partielle (24) à l'intérieur de laquelle, pendant la période de non-introduction du liquide, la pièce de raccordement (3), traversant la lumière dirigée vers la cloison nasale, est fixée, assujettie en place mais amovible, et
- dans la zone partielle (24), dans la section associée de la surface de paroi interne (surface de positionnement 24), est ménagé un pas de vis (26) qui peut être amené en prise active avec la surface d'appui (13) de la pièce de raccordement (3).

5.- Dispositif suivant la revendication 4, caractérisé en ce que sur la surface de positionnement (24) opposée à la lumière postérieure (23) du creux (21) est prévu un ergot de guidage (25) destiné à un évidement associé de la pièce de raccordement (3).

6.- Dispositif suivant l'une quelconque des revendications 1 à 5, caractérisé en ce que la pièce de raccordement (3) a la forme d'un raccord de sonde souple conventionnel dont la surface d'appui (13) a la forme d'une collerette.

7.- Dispositif suivant l'une quelconque des revendications 1 à 6, caractérisé en ce que la surface de la pièce de positionnement (1, 20) présente une rugosité faible et la pièce de positionnement (1, 20) est faite d'une matière physiologiquement tolérable.

EP 0 204 986 B1

FIG. 1

FIG. 2

FIG. 3

FIG. 4

FIG. 5

FIG. 6